# FASCICULE DE BREVET EUROPEEN

(11) **EP 0 472 457 B1**
(45) Date de publication et mention de la délivrance du brevet: **02.11.1994**
(21) Numéro de dépôt: 91402259.5
(22) Date de dépôt: 19.08.1991
(51) Int. Cl.: B67D 5/02, B67B 7/48

(54) **Appareil de vidange et de rinçage de flacons non récupérables contenant un produit toxique**
Vorrichtung zur Entleerung und Spülung von ein giftiges Produkt enthaltenden Wegwerfbehältern
Purging and flushing apparatus for disposable bottles containing a toxic product

(30) Priorité: 20.08.1990 FR 9010477
(43) Date de publication de la demande: 26.02.1992
(73) Titulaire: COGEMA COMPAGNIE GENERALE DES MATIERES NUCLEAIRES, F-78141 Velizy-Villacoublay (FR)
(72) Inventeur: Cauquil,Gérard, F-30200 Bagnol-sur-Cèze (FR); Chazot, Henri, F-30133 Les Angles (FR)
(74) Mandataire: Poulin, Gérard

(56) Documents cités:
- EP-A- 0 249 377
- FR-A- 1 097 955
- US-A- 3 685 694
- US-A- 3 993 221
- US-A- 4 192 438
- US-A- 4 396 340

## Description

L'invention concerne un appareil permettant de vidanger et, si nécessaire, de rincer des flacons non réutilisables contenant un produit toxique tel qu'une solution de faible activité comportant des émetteurs gamma et ayant servi à effectuer certaines analyses.

Pour analyser les émetteurs gamma contenus dans une solution de faible activité, on procède fréquemment au comptage d'une certaine quantité de liquide introduite préalablement dans un flacon muni d'un opercule et fermé par un bouchon à vis. Ce flacon est habituellement réalisé en une matière plastique telle que du polyéthylène. Lorsque l'analyse est terminée, les flacons doivent être vidés, rincés, puis évacués de façon à être stockés dans des fûts aptes à recevoir les déchets.

Actuellement, cette opération nécessite, pour chacun des flacons, de dévisser le bouchon, enlever l'opercule, vidanger le flacon, le rincer, le vidanger à nouveau, puis l'évacuer dans un fût. Ces différentes tâches, effectuées manuellement à l'intérieur de la cellule plusieurs dizaines de fois par semaine, sont particulièrement fastidieuses et entraînent une contamination générale du poste de travail.

On connait aussi du document US-A-4 192 438 un dispositif conçu pour extraire un gaz radioactif d'un flacon obturé par une membrane perforable. Ce dispositif comprend une aiguille à deux parois coaxiales, et des moyens pour injecter un gaz porteur par le passage annulaire de l'aiguille, de façon à évacuer le gaz radioactif mélangé à ce gaz porteur, par le passage central de l'aiguille.

L'invention a précisément pour objet un appareil permettant d'effectuer la vidange, et si nécessaire, le rinçage de tels flacons d'une manière automatisée et sans entraîner de contamination notable du poste de travail.

Pour parvenir à ce résultat, il est proposé un appareil de vidange de flacons contenant un liquide et comportant au moins une cloison perforable, comprenant :
- une aiguille à deux parois coaxiales, apte à être enfoncée dans le flacon au travers de la cloison perforable, les parois coaxiales de l'aiguille délimitant un passage central et un passage annulaire, et
- des moyens pour injecter un gaz sous pression dans l'aiguille par un premier desdits passages, de façon à vidanger le liquide par l'autre passage,
caractérisé par le fait qu'il comprend de plus :
- des moyens pour injecter un liquide de rinçage sous pression par le premier passage, de façon à assurer le rinçage des flacons vidangés ;
- un barillet rotatif apte à tourner autour d'un axe vertical et comportant au moins trois alvéoles aptes à recevoir chacune un flacon retourné ; et
- des moyens pour entraîner le barillet en rotation, de façon à faire défiler successivement chaque alvéole devant un poste de chargement, un poste de vidange comportant des moyens pour déplacer ladite aiguille entre une position haute d'attente et une position basse de vidange, et un poste d'évacuation.

En perforant le fond de chacun des flacons à l'aide de l'aiguille à double paroi, puis en injectant un gaz tel que de l'air sous pression dans le flacon au travers de l'aiguille, on évacue de façon automatique et sans risque de contamination les solutions de faible activité contenues dans les flacons.

En équipant, de plus, cet appareil de moyens pour injecter un liquide de rinçage sous pression par le premier passage, on peut ensuite effectuer le rinçage de chacun des flacons dans les mêmes conditions de confort et de sécurité. Après rinçage, le liquide de rinçage contenu dans chaque flacon est vidangé en injectant à nouveau un gaz sous pression dans chaque flacon au travers de l'aiguille.

De préférence, le premier passage par lequel sont injectés le gaz sous pression et, le cas échéant, le liquide de rinçage sous pression, est le passage annulaire délimité entre les parois coaxiales de l'aiguille.

L'aiguille comprend alors, avantageusement, une paroi tubulaire intérieure, une paroi tubulaire extérieure et une pointe de perforation fixée à l'une au moins de ces parois, la paroi tubulaire extérieure comportant au moins un perçage situé dans une zone éloignée de ladite pointe de perforation, et la pointe de perforation comportant au moins un perçage débouchant dans le passage central délimité par la paroi tubulaire intérieure.

Avantageusement, l'appareil selon l'invention comprend, de plus, un panier rotatif apte à tourner autour d'un axe vertical parallèle à l'axe du barillet, ce panier comportant des colonnes tubulaires verticales dans lesquelles peuvent être placés plusieurs flacons retournés, une platine horizontale fixe placée en dessous du panier et sur laquelle reposent les flacons, et des moyens pour entraîner le panier en rotation, de façon à amener successivement chaque colonne tubulaire au-dessus d'une ouverture formée dans la platine, ladite ouverture définissant le poste de chargement du barillet, placé en dessous de ladite platine.

Un mode de réalisation préféré de l'invention va à présent être décrit, à titre d'exemple non limitatif, en se référant aux dessins annexés, dans lesquels :
- la figure 1 est une vue de face, en coupe partielle, représentant de façon schématique un appareil de vidange et de rinçage de flacons conforme à l'invention ;
- la figure 2 est une vue à plus grande échelle, en coupe partielle, représentant le poste de vidange et de rinçage des flacons dans l'appareil de la figure 1 ; et
- la figure 3 est une vue en coupe longitudinale illustrant à plus grande échelle l'aiguille à double paroi équipant le poste de vidange et de rinçage des flacons représenté sur la figure 2.

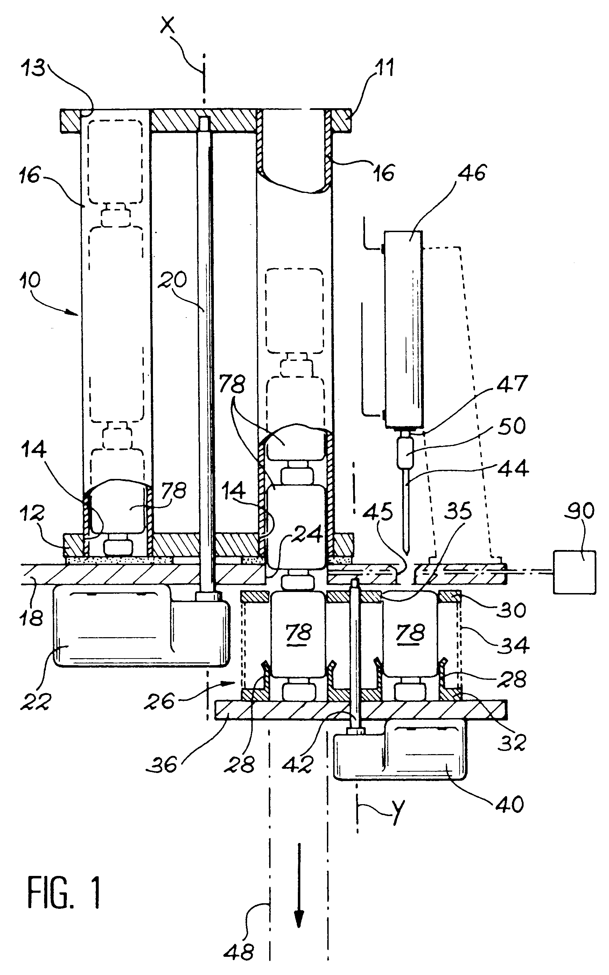

Sur la figure 1, la référence 10 désigne de façon générale un panier rotatif présentant un axe vertical X. Ce panier 10 comporte un plateau supérieur horizontal 11 et un plateau inférieur horizontal 12 dans lesquels sont formées des ouvertures circulaires respectives 13, 14, par exemple au nombre de huit, régulièrement réparties autour de l'axe vertical X. Ces ouvertures circulaires 13 et 14 sont reliées deux à deux, par des colonnes tubulaires 16 d'axes verticaux, fixées sur les plateaux 12.

Le panier rotatif 10 repose sur une platine supérieure horizontale, 18 placée immédiatement en dessous du plateau 12. Les plateaux 11 et 12 sont solidaires d'un arbre vertical 20 disposé selon l'axe X et qui traverse la platine 18. Un ensemble moteur 22, placé en dessous de la platine 18, assure l'entraînement en rotation du panier 10 par l'intermédiaire de l'arbre 20.

Une ouverture circulaire 24, sensiblement de même diamètre que les ouvertures circulaires 13 et 14, est formée dans la platine 18, en un emplacement tel que chacune des ouvertures circulaires 14 peut être amenée successivement en face de cette ouverture circulaire 24 lors de la rotation du panier 10.

L'appareil de vidange et de rinçage illustré sur la figure 1 comprend de plus un barillet rotatif désigné de façon générale par la référence 26. Ce barillet rotatif comporte un axe vertical Y décalé par rapport à l'axe X au-delà de l'axe de l'ouverture circulaire 24, de telle sorte que ces trois axes se trouvent situés dans un même plan vertical.

Le barillet rotatif 26 est placé en dessous de la platine 18 et il comporte trois alvéoles matérialisées par des réceptacles tubulaires 28 d'axes verticaux, régulièrement répartis autour de l'axe Y. La distance séparant chaque alvéole de l'axe Y est telle que ces alvéoles peuvent être amenées successivement dans le prolongement de l'ouverture circulaire 24 par une rotation du barillet 26.

Dans l'exemple de réalisation illustré schématiquement sur la figure 1, le barillet 26 comprend une plaque supérieure horizontale 30 et une plaque inférieure horizontale 32 reliées entre elles par une virole périphérique 34. La plaque inférieure horizontale supporte les réceptacles 28 et la plaque supérieure horizontale 30 comporte une ouverture circulaire 35 au droit de chacun de ces réceptacles.

Le barillet 26 repose sur une platine inférieure horizontale 36 solidarisée de la platine supérieure 18 par exemple par des colonnettes (non représentées). L'entraînement en rotation du barillet 26 autour de l'axe vertical Y est assuré par un ensemble moteur 40 monté en dessous de la platine 36 et dont l'arbre de sortie vertical 42, disposé selon l'axe Y, traverse la platine 36 pour être solidarisé des plaques 30 et 32 du barillet 26.

Lorsque l'un des réceptacles 28 du barillet 26 se trouve placé dans l'alignement de l'ouverture circulaire 24 formée dans la platine 18 (cette ouverture constituant le poste de chargement du barillet 26), les deux autres alvéoles 28 du barillet 26 se trouvent respectivement en face d'un poste de vidange et de rinçage des flacons et en face d'un poste d'évacuation des flacons. Ces deux derniers postes sont décalés de façon à se trouver au-delà de la périphérie extérieure du panier 10.

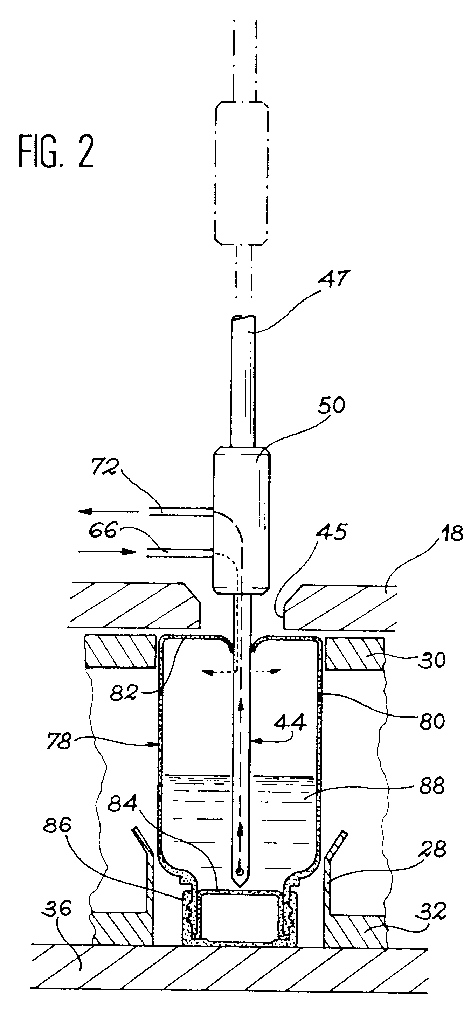

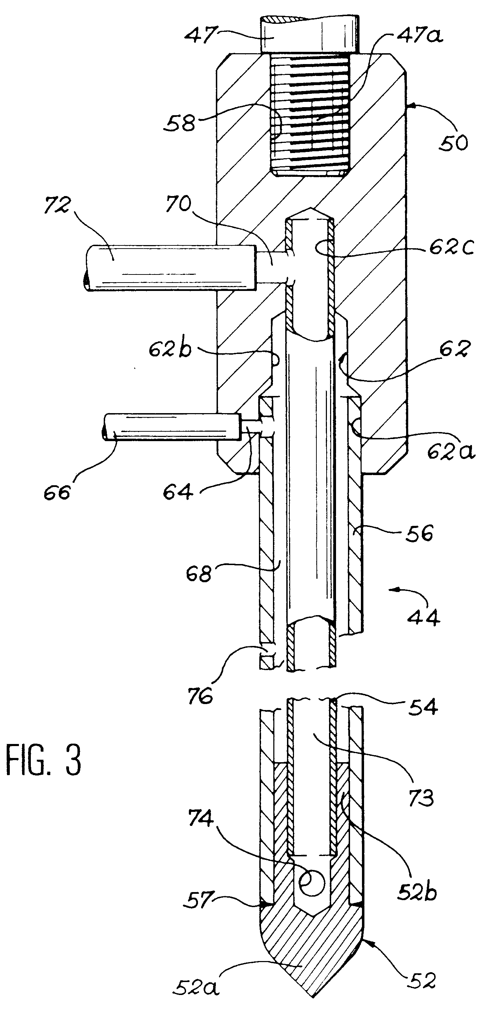

Le poste de vidange et de rinçage des flacons comprend principalement, selon une caractéristique essentielle de l'invention, une aiguille à double paroi 44 qui sera décrite plus en détail par la suite en se référant aux figures 2 et 3. Cette aiguille 44 est disposée selon un axe vertical sensiblement aligné avec l'axe du réceptacle 28 situé en face du poste de vidange et de rinçage des flacons. Elle est supportée par la tige 47 d'un vérin porte-aiguille 46 qui permet de la déplacer selon son axe entre une position haute d'attente, illustrée sur la figure 1, et une position basse de vidange, illustrée sur la figure 2.

Dans sa position haute d'attente, l'aiguille à double paroi 44 est située en totalité au-dessus de la platine 18, alors que, lorsqu'elle occupe sa position basse de vidange, l'aiguille 44 traverse une ouverture 45 formée à cet effet dans la platine 18 et pénètre dans le flacon reçu dans le réceptacle 28 correspondant, comme on le verra plus en détail dans la suite de la description.

Le poste d'évacuation en face duquel se trouve la troisième alvéole 28 du barillet 26 est matérialisé quant à lui par une manchette tubulaire 48 placée en dessous d'une ouverture circulaire (non représentée) formée à cet endroit dans la platine 36. Les flacons sont automatiquement évacués par gravité lorsqu'ils parviennent en face de cette ouverture après une rotation du barillet 26. L'extrémité opposée de la manchette 48 débouche directement dans un fût de stockage de déchets (non représenté), après avoir traversé la paroi de la cellule dans laquelle est placé l'appareil.

Comme l'illustre plus en détail la figure 3, l'aiguille 44 à double paroi comprend une pièce de tête 50, une pointe 52 et deux parois tubulaires coaxiales constituées respectivement par une paroi tubulaire intérieure 54 et une paroi tubulaire extérieure 56.

A son extrémité supérieure, la pièce de tête 50 comporte un trou taraudé 58, par lequel l'aiguille 44 est fixée à l'extrémité filetée 47a de la tige 47 du vérin porte-aiguille 46. A son extrémité inférieure, la pièce de tête 50 comporte un alésage étagé 62, aligné avec le trou taraudé 58. Cet alésage 62 comporte une partie inférieure 62a, de relativement grand diamètre, dans laquelle est reçue l'extrémité supérieure de la paroi tubulaire extérieure 56, une partie intermédiaire 62b de diamètre moyen, et une partie supérieure 62c de relativement petit diamètre, dans laquelle est reçue l'extrémité haute de la paroi tubulaire intérieure 54.

La pièce de tête 50 comporte un premier raccord relié par un passage 64 à la partie inférieure 62a ou à la partie intermédiaire 62b de l'alésage étagé 62. Sur ce premier raccord est monté un tube 66 permettant d'injecter un gaz sous pression tel que de l'air comprimé ou un liquide de rinçage sous pression tel que de l'eau dans un espace annulaire 68 délimité entre les parois tubulaires 54 et 56.

La pièce de tête 50 comporte aussi un second raccord relie par un passage 70 à la partie supérieure de l'alésage étagé 62 et sur lequel est monté un tube 72 permettant d'évacuer les différents fuides hors des flacons, par un passage central 73 formé dans la paroi tubulaire intérieure 54.

La pointe 52 de l'aiguille 44 forme à son extrémité inférieure un cône de perforation 52a permettant à l'aiguille de traverser la paroi en matière plastique des flacons. La pointe 52 comporte de plus une partie supérieure tubulaire 52b autour de laquelle est montée la paroi tubulaire extérieure 56 et dans laquelle est reçue la paroi tubulaire intérieure 54. La fixation de la pointe 52 à l'extrémité de la paroi tubulaire extérieure 56 est avantageusement effectuée au moyen d'une soudure argon 57.

Au moins un perçage 74 traverse radialement l'extrémité inférieure de la paroi tubulaire extérieure 56 et la partie supérieure tubulaire 52b de la pointe 52, de façon à déboucher en dessous de l'extrémité inférieure de la paroi tubulaire intérieure 54.

On observe également sur la figure 3 que la paroi tubulaire extérieure 56 est également traversée, dans sa partie haute située à proximité de la pièce de tête 50, par au moins un perçage 76 permettant au passage annulaire 68 de communiquer avec l'extérieur.

Le tube d'introduction 66 est relié, à son extrémité opposée à l'aiguille 44, à un circuit de gaz sous pression tel que de l'air comprimé à environ 2 bars par l'intermédiaire d'une première électrovanne (non représentée), et à un circuit de liquide de rinçage sous pression tel que de l'eau à environ 3 bars par l'intermédiaire d'une deuxième électrovanne (non représentée). Le tube d'évacuation 72 est relié quant à lui à un circuit d'évacuation des effluents.

Le fonctionnement automatisé de l'appareil de vidange et de rinçage de flacons qui vient d'être décrit est avantageusement géré par un automate programmable tel que l'automate SIEMENS SR501-U, grâce aux informations fournies par différents capteurs tels que des capteurs optiques, selon des techniques classiques bien connues de l'homme du métier.

Le fonctionnement de cet appareil de vidange et de rinçage va à présent être décrit en se référant notamment aux figures 1 et 2.

Sur la figure 2, on a représenté à plus grande échelle l'un des flacons 78 dont la vidange et le rinçage peuvent être effectués à l'aide de l'appareil selon l'invention. Ce flacon 78 comporte un corps 80, de section circulaire, comportant notamment un fond plat 82, et qui est réalisé en un matériau apte à être perforé par la pointe de l'aiguille 44, tel que du polyéthylène. L'embouchure du corps 80 du flacon 78 est obturée intérieurement par un opercule 84 et extérieurement par un bouchon vissé 86. Chacun des flacons est rempli d'une solution 88 qui doit être vidangée et nécessite, dans l'exemple décrit, le rinçage du flacon avant que ce dernier ne soit évacué en fût.

Comme l'illustre schématiquement la figure 1, les flacons dont on désire réaliser la vidange et le rinçage sont empilés, par exemple par quatre dans l'exemple représenté, dans chacune des colonnes tubulaires verticales 16 du panier rotatif 10. Plus précisément, les flacons 78 sont retournés avant d'être placés dans les colonnes 16, de telle sorte que leurs fonds 82 soient tournés vers le haut.

Lorsque toutes les colonnes 16 sont remplies de flacons 78, l'opération de vidange et de rinçage de ces flacons peut commencer. Cette opération, réalisée de façon totalement automatique, permet de traiter en moyenne environ 60 flacons à l'heure sans qu'aucune manutention n'ait à être réalisée afin d'assurer le conditionnement définitif des flacons dans les fûts.

Dès que l'appareil est actionné, le panier 10 est entraîné en rotation autour de son axe par l'ensemble moteur 22. Cette rotation se poursuit jusqu'à ce que l'une des colonnes tubulaires verticales 16 arrive au-dessus de l'ouverture circulaire 24. Les flacons 78 qui se trouvent dans cette colonne chutent alors par gravité, de telle sorte que le flacon inférieur se trouve reçu dans l'un des réceptacles 28 du barillet 26. Cette chute est détectée par un capteur à fibre optique 90 placé au niveau de la platine 18 et qui stoppe automatiquement l'ensemble moteur 22.

Le barillet 26 est alors entraîné en rotation par l'ensemble moteur 40, jusqu'à l'arrivée du flacon 78 au droit du poste de vidange et de rinçage des flacons. Lorsque le barillet se trouve dans cette position, détectée par un capteur électrique (non représenté), un autre flacon 78 chute par gravité dans le réceptacle 28 qui se trouve alors située en dessous de l'ouverture circulaire 24.

Un cycle de vidange et de rinçage du flacon 78 situé au droit du poste de vidange et de rinçage est alors initié. Ce cycle va à présent être décrit en se référant plus précisément à la figure 2.

Au début de ce cycle, le vérin porte-aiguille 46 est actionné automatiquement dans le sens de la descente de l'aiguille 44 à double paroi. Au cours de cette descente, la pointe 52 de l'aiguille perfore le fond 82 du flacon et l'aiguille s'enfonce dans ce dernier jusqu'à ce que la pointe arrive à proximité de l'opercule 84, dans la position basse de vidange illustrée sur la figure 2. Cette position basse est détectée par un capteur magnétique (non représenté). Les perçages 76 se trouvent alors à l'intérieur du flacon 78 légérement en dessous du fond 82 et au-dessus de la solution 88.

La vidange de la solution 88 contenue dans le flacon est alors effectuée en injectant de l'air comprimé (environ deux bars) au-dessus de cette solution par le tube 66, le passage annulaire 68 et les perçages 76. L'air comprimé chasse la solution 88 au travers du perçage 74, du passage central 73 et du tube 72.

Dans l'exemple décrit, le flacon 78 est ensuite rincé en injectant de l'eau de rinçage par le tube 66, le passage annulaire 68 et les perçages 76. L'eau sous pression (environ 3 bars) est pulvérisée à l'intérieur du flacon, ce qui optimise le ruissellement sur les parois et, par conséquent, l'efficacité du rinçage.

L'eau de rinçage est ensuite chassée du flacon par une deuxième injection d'air comprimé effectuée dans les mêmes conditions que l'injection ayant servi à vidanger le flacon, mais pendant une période plus longue.

Lorsque la pression à l'intérieur du flacon est revenue à la pression atmosphérique, ce qui supprime les projections lors de la sortie de l'aiguille 44, celle-ci est ramenée vers sa position haute d'attente illustrée sur la figure 1 par le vérin 46. Dès qu'un contact magnétique (non représenté) détecte le retour de l'aiguille 44 dans cette position haute, l'ensemble moteur 40 est à nouveau actionné afin d'amener le flacon vidangé et rincé au droit du poste d'évacuation et d'amener le flacon suivant au droit du poste de vidange et de rinçage. Le flacon vidangé et rincé est alors évacué automatiquement par gravité dans la manchette tubulaire 48 et un nouveau cycle de vidange et de rinçage est initié sur le flacon suivant. Dans cette position, un troisième flacon contenu dans la colonne tubulaire verticale 16 qui se trouve au-dessus de l'ouverture circulaire 24 chute dans le réceptacle 28 placé en dessous de cette ouverture.

Dès que le capteur optique 90 placé au niveau de l'ouverture circulaire 24 détecte que la colonne 16 qui se trouve au-dessus de cette ouverture est vide, l'ensemble moteur 22 est automatiquement actionné afin d'amener une autre colonne 16 au-dessus de cette ouverture circulaire.

Le cycle se poursuit ainsi jusqu'à ce que l'ensemble des colonnes tubulaires verticales 16 soit vide. L'appareil s'arrête alors automatiquement grâce aux informations fournies par un capteur électrique (non représenté) assurant le comptage des colonnes tubulaires 16.

L'appareil selon l'invention permet, comme on vient de le voir, de réaliser de façon entièrement automatique la vidange et le rinçage des flacons sans qu'il soit nécessaire d'en dévisser le bouchon ni d'en enlever l'opercule, ce qui serait très difficile à faire en automatique.

Bien entendu, l'invention n'est pas limitée au mode de réalisation qui vient d'être décrit à titre d'exemple, mais en couvre toutes les variantes. Ainsi, le panier 10 et le barillet 26, qui constituent des moyens particuliers pour faire défiler de façon automatique des flacons à vidanger et à rincer devant l'aiguille à double paroi au moyen de laquelle ces opérations sont réalisées, peuvent dans certains cas être supprimés, modifiés ou remplacés par des moyens équivalents tels qu'un transporteur pas à pas. Par ailleurs, lorsque la nature du liquide contenu dans le flacon à vidanger le permet, l'opération de rinçage peut être supprimée.

## Revendications

1. Appareil de vidange de flacons contenant un liquide et comportant au moins une cloison perforable, comprenant :
- une aiguille (44) à deux parois coaxiales, apte à être enfoncée dans le flacon au travers de la cloison perforable, les parois coaxiales (54, 56) de l'aiguille délimitant un passage central (73) et un passage annulaire (68) ;
- des moyens (66) pour injecter un gaz sous pression dans l'aiguille par un premier (68) desdits passages, de façon à vidanger le liquide par l'autre passage (73),
caractérisé par le fait qu'il comprend de plus :
- des moyens (66) pour injecter un liquide de rinçage sous pression par le premier passage (68), de façon à assurer le rinçage des flacons vidangés ;
- un barillet rotatif (26) apte à tourner autour d'un axe vertical et comportant au moins trois alvéoles (28) aptes à recevoir chacune un flacon retourné ; et
- des moyens (40) pour entraîner le barillet en rotation, de façon à faire défiler successivement chaque alvéole devant un poste de chargement, un poste de vidange comportant des moyens (46) pour déplacer ladite aiguille (44) entre une position haute d'attente et une position basse de vidange, et un poste d'évacuation.

2. Appareil selon la revendication 1, caractérisé par le fait que le premier passage est le passage annulaire (68) délimité entre les parois coaxiales de l'aiguille.

3. Appareil selon la revendication 2, caractérisé par le fait que l'aiguille (44) comprenant une paroi tubulaire intérieure (54), une paroi tubulaire extérieure (64) et une pointe de perforation (52) fixée à l'une au moins de ces parois, la paroi tubulaire extérieure comporte au moins un perçage (76) situé dans une zone éloignée de ladite pointe de perforation, et la pointe de perforation comporte au moins un perçage (74) débouchant dans le passage central (73) délimité par la paroi tubulaire intérieure.

4. Appareil selon l'une quelconque des revendications 1 à 3, caractérisé par le fait qu'il comprend de plus un panier rotatif (10) apte à tourner autour d'un axe vertical parallèle à l'axe du barillet, ce panier comportant des colonnes tubulaires verticales (16) dans lesquelles peuvent être placés plusieurs flacons retournés, une platine horizontale fixe (18) placée en dessous du panier et sur laquelle reposent les flacons, et des moyens (22) pour entraîner le panier en rotation, de façon à amener successivement chaque colonne tubulaire (16) au-dessus d'une ouverture (24) formée dans la platine, ladite ouverture définissant le poste de chargement du barillet (26), placé en dessous de ladite platine.

## Claims

1. Apparatus for emptying flasks containing a liquid and comprising at least one perforatable partition, comprising a needle (44) having two coaxial walls, which can be inserted in the flask through the perforatable partition, the coaxial walls (54, 56) of the needle defining a central passage (73) and an annular passage (68) and means (66) for injecting a pressurized gas into the needle by a first (68) of the said passages, so as to empty the liquid through the other passage (73), characterized in that it also comprises means for injecting a pressurized rinsing liquid through the first passage (68), so as to rinse the empty bottles, a rotary drum (26) able to rotate about a vertical axis and having at least three cavities (28), each being able to receive an inverted bottle and means (40) for rotating the drum so as to successively pass each cavity in front of a loading station, an emptying station having means (46) for moving the said needle (44) between an upper waiting position and a lower emptying position, and a discharge station.

2. Apparatus according to claim 1, characterized in that the first passage is the annular passage (68) defined between the coaxial walls of the needle.

3. Apparatus according to claim 2, characterized in that the needle (44) has an inner tubular wall (54), an outer tubular wall (64) and a perforating tip (52) fixed to at least one of these walls, the outer tubular wall having at least one opening (76) in an area remote from said perforating tip, and the perforating tip has at least one opening (74) issuing into the central passage (73) defined by the inner tubular wall.

4. Apparatus according to any one of the claims 1 to 3, characterized in that it also comprises a rotary basket (10) able to rotate about a vertical axis parallel to the drum axis, said basket incorporating vertical tubular columns (16) in which can be placed several inverted bottles, a fixed horizontal panel (18) placed below the basket and on which rests the said bottles, and means (22) for rotating the basket, so as to successively bring each tubular column (16) above an opening (24) formed in the panel, said opening defining the loading station of the barrel (26) placed below the said panel.

## Patentansprüche

1. Vorrichtung zur Entleerung von Behältern, die eine Flüssigkeit enthalten und wenigstens eine perforierbare Wand aufweisen, umfassend:
- eine Nadel (44) mit zwei koaxialen Wänden, die in die Flasche hineingstochen werden kann durch die perforierbare Wand, wobei die koaxialen Wände (54, 56) der Nadel einen zentralen Durchgang (73) und einen ringförmigen Durchgang (68) begrenzen;
- Einrichtungen (66), um über einen ersten (68) der genannten Durchlässe ein Druckgas in den Behälter einzupressen, um die Flüssigkeit durch den anderen Durchgang (73) zu entleeren;
**dadurch gekennzeichnet,** daß sie außerdem umfaßt:
- Einrichtungen (66), um über den ersten Durchgang (68) eine Reinigungsflüssigkeit unter Druck einzuspritzen, um die Reinigung der geleerten Behälter sicherzustellen;
- eine Drehtrommel (26), drehbar um eine Vertikalachse und wenigstens drei Aufnahmen (28) umfassend, jede geeignet, einen umgedrehten Behälter aufzunehmen; und
- Einrichtungen (40) um die Trommel in eine Drehbewegung zu versetzen, um sukzessive jede Aufnahme bei einer Ladestation vorbeizuführen, eine Entleerungsstation, die Einrichtungen (46) umfaßt, um die genannte Nadel (44) zwischen einer oberen Wartestellung und einer unteren Reinigungsstellung zu verschieben, und eine Beseitigungsstation.

2. Vorrichtung nach Anspruch 1, dadurch gekennzeichnet, daß der erste Durchgang der zwischen den koaxialen Wänden der Nadel begrenzte ringförmige Durchgang (68) ist.

3. Vorrichtung nach Anspruch 2, dadurch gekennzeichnet, daß die Nadel (44) eine röhrenförmige Innenwand (54), eine röhrenförmige Außenwand (64) und eine Perforierungsspitze (52) umfaßt, die an wenigstens einer dieser Wände befestigt ist, wobei die röhrenförmige Außenwand wenigstens eine Bohrung (76) enthält, angebracht in einem von der genannten Perforierungspitze entfernten Bereich, und die Perforierungsspitze wenigstens eine Bohrung (74) enthält, die in dem zentralen Durchgang (73) mündet, begrenzt durch die röhrenförmige Innenwand.

4. Vorrichtung nach einem der Ansprüche 1 bis 3, dadurch gekennzeichnet, daß sie außerdem einen Drehkorb (10) umfaßt, der um eine zur Achse der Trommel parallele Vertikalachse dreht, wobei dieser Korb röhrenförmige Vertikalsäulen (16) umfaßt, in denen mehrere umgedrehte Behälter untergebracht werden können, eine feste Horizontalplatte (18), angeordnet unterhalb des Korbs, auf der die Behälter aufliegen, und Einrichtungen (22), um den Korb in eine Drehbewegung zu versetzen, um sukzessive jede röhrenförmige Säule (16) über eine in der Platte ausgebildete Öffnung (24) zu bewegen, wobei die genannte Öffnung die Ladestation der Trommel (26) definiert, die unterhalb der genannten Platte angeordnet ist.
